# FASCICULE DE BREVET EUROPEEN

(11) **EP 1 148 881 B1**
(45) Date de publication et mention de la délivrance du brevet: **09.08.2006**
(21) Numéro de dépôt: 00900637.0
(22) Date de dépôt: 21.01.2000
(51) Int. Cl.: A61K 31/5025, A61K 31/5377, A61P 25/00, A61P 9/00, A61P 13/00

(54) **UTILISATION DE DERIVES DE PYRIDAZINO ¬4,5-(b)|-INDOLE-1-ACETAMIDE POUR LA PREPARATION DE MEDICAMENTS DESTINES AUX MALADIES LIEES AU DYSFONCTIONNEMENT DES RECEPTEURS DE TYPE PERIPHERIQUE AUX BENZODIAZEPINES**
VERWENDUNG VON PYRIDAZINO ¬4,5-(b)|-INDOLE-1-ACETAMIDE DERIVATEN ZUR HERSTELLUNG EINES ARZNEIMITTELS ZUR BEHANDLUNG VON DURCH PERIPHERE BENZODIAZEPINREZEPTOREN-STÖRUNGEN VERURSACHTE KRANKHEITEN
USE OF PYRIDAZINO ¬4,5-(b)|-INDOLE-1-ACETAMIDE DERIVATIVES FOR PREPARING MEDICINES FOR TREATING DISEASES RELATED TO THE DYSFUNCTION OF PERIPHERAL BENZODIAZEPIN RECEPTORS

(30) Priorité: 26.01.1999 FR 9900806
(43) Date de publication de la demande: 31.10.2001
(73) Titulaire: Sanofi-Aventis, 75013 Paris (FR)
(72) Inventeur: FERZAZ, Badia, F-92160 Antony (FR); BENAVIDES, Jésus, F-92290 Chatenay-Malabry (FR); MARGUET, Frank, F-91370 Verrières le Buisson (FR); FROISSANT, Jacques, F-41160 Morée (FR); MARABOUT, Benoît, F-91380 Chilly Mazarin (FR); EVANNO, Yannick, F-75013 Paris (FR); SEVRIN, Mireille, F-75014 Paris (FR); JANIAK, Philip, F-91160 Gif-sur-Yvette (FR)
(74) Mandataire: Ludwig, Jacques
(86) Numéro de dépôt international: PCT/FR2000/000135
(87) Numéro de publication internationale: WO 2000/044384

(56) Documents cités:
- WO-A-98/15552
- WO-A-99/06406
- DE-A- 3 121 137
- GB-A- 2 290 292
- ALI, M. I. ET AL: "Reactions with 1-alkylindole-2-carboxy-3-acetic acid anhydrides" INDIAN J. CHEM., SECT. B (1977), 15(1), 64-6, XP002118938

## Description

Utilisation de dérivés de pyridazino[4,5-*b*]indole-1-acétamide pour la préparation de médicaments destinés aux maladies liées au dysfonctionnement des récepteurs de type périphérique aux benzodiazépines.

Dans le cadre de la recherche de composés pouvant favoriser la régénération des axones des cellules nerveuses périphériques après lésion, il a été identifié, parmi les composés de la demande de brevet internationale PCT/FR98/01667, une sous-classe de composés de formule générale (I) 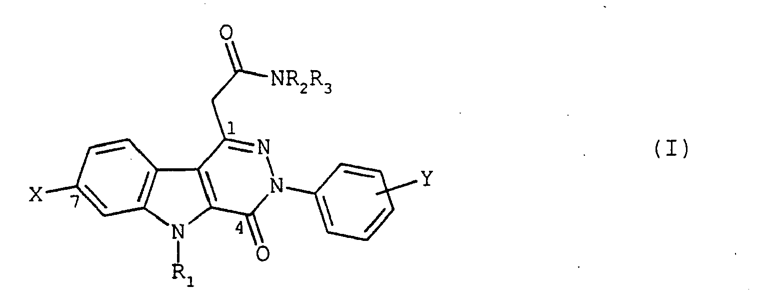 dans laquelle
X représente un atome d'halogène,
Y représente un ou plusieurs atomes ou groupes choisis parmi l'hydrogène, les halogènes et les groupes hydroxy, méthyle, méthoxy et nitro,
R₁ représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle ou morpholinyle.

Ces composés possèdent une forte affinité pour les récepteurs de type périphérique aux benzodiazépines (sites p, ou PBR), et certains induisent, notamment, une diminution de la perte neuronale dans le noyau facial après section du nerf facial. Ils présentent aussi des effets cardio- et rénoprotecteurs.

Un composé particulièrement intéressant pour l'utilisation selon l'invention est, par exemple, le 7-chloro-*N*,*N*,5-triméthyl-4-oxo-3-phényl-3,5-dihydro-4*H*-pyridazino[4,5-*b*]-indole-1-acétamide.

Ce dernier peut-être préparé selon le mode opératoire suivant, donné à titre d'exemple.

Exemple (Composé N°1 du tableau qui suit).

### 1. 6-Chloro-1-méthyl-1H-indole-2-carboxylate d'éthyle.

On agite pendant 2 h à température ambiante une suspension de 1,8 g (45 mmoles) d'hydrure de sodium à 60% (préalablement lavé à l'éther de pétrole) et de 8,0 g (35,8 mmoles) de 6-chloro-1*H*-indole-2-carboxylate d'éthyle dans 80 ml de N,N-diméthylformamide, on ajoute ensuite 2,8 ml (45 mmoles) de iodométhane et on agite le mélange à température ambiante pendant 4 H.
On ajoute 5 ml d'éthanol absolu et on évapore le solvant sous pression réduite. On reprend le résidu par de l'eau et on extrait le mélange par du dichlorométhane, on sèche la phase organique, on filtre, on évapore le solvant sous pression réduite et on purifie le résidu par chromatographie sur colonne de gel de silice.
On isole 8,5 g (35,7 mmoles) d'un composé blanc cristallin. Point de fusion : 75,5-76,5°C.

### 2. 6-Chloro-2-(éthoxycarbonyl)-1-méthyl-α-oxo-1H-indole-3-acétate d'éthyle.

A une solution de 4 ml (36 mmoles) de chlorooxoacétate d'éthyle dans 100 ml de 1,2-dichloroéthane, on ajoute 4 ml (36,4 mmoles) de tétrachlorure de titane. On agite le mélange réactionnel pendant 30 min à température ambiante, puis on ajoute 7,8 g (32,8 mmoles) de 6-chloro-1-méthyl-1*H-*indole-2-carboxylate d'éthyle et on agite le mélange réactionnel pendant 4 h à température ambiante.
On refroidit le milieu, on ajoute 200 ml de dichlorométhane et 100 ml d'eau. On décante la phase organique, on la lave à l'eau, on la sèche sur sulfate de sodium, on la filtre, on concentre le filtrat sous pression réduite et on purifie le résidu par chromatographie sur colonne de gel de silice.
On isole 9,4 g (29,0 mmoles) de produit.
Point de fusion : 94-95°C.

### 3. 7-Chloro-5-méthyl-4-oxo-3-phényl-3,5-dihydro-4H-pyridazino[4,5-b]indole-1-carboxylate d'éthyle.

A une solution de 4,6 g (13,6 mmoles) de 6-chloro-2-(éthoxycarbonyl)-1-méthyl-α-oxo-1*H*-indole-3-acétate d'éthyle dans 120 ml d'acide acétique, on ajoute à température ambiante 4 ml (40,6 mmoles) de phénylhydrazine.
On agite le mélange réactionnel pendant 30 min à température ambiante, puis pendant 4 h au reflux.
On refroidit le milieu, on ajoute 350 ml de dichlorométhane et 100 ml d'eau. On décante la phase organique, on la lave avec une solution aqueuse saturée d'hydrogénocarbonate de sodium, puis avec de l'eau, on la sèche sur sulfate de sodium, on la filtre, on la concentre sous pression réduite et on purifie le résidu par chromatographie sur colonne de gel de silice.
On isole 4,1 g (10,7 mmoles) de produit.
Point de fusion : 216-218,5°C.

### 4. 7-Chloro-1-(hydroxyméthyl)-5-méthyl-3-phényl-3,5-dihydro-4H-pyridazino[4,5-b]indol-4-one.

A une solution de 4,04 g (10,6 mmoles) de 7-chloro-5-méthyl-4-oxo-3-phényl-3,5-dihydro-4*H*-pyridazino[4,5-b]indole-1-carboxylate d'ethyle dans 150 ml de tétrahydrofuranne, on ajoute 2,5 g (66,1 mmoles) de borohydrure de sodium. Sous agitation, on ajoute progressivement 2,25 ml de méthanol, puis on chauffe le mélange au reflux durant 5 h.
On verse le mélange sur une solution glacée d'acide chlorhydrique 1 M, on isole un insoluble par filtration sur verre fritté, que l'on lave à l'eau et à l'éther diéthylique, puis on le sèche.
On isole 3,3 g (9,7 mmoles) de composé sous forme d'un solide blanc qu'on utilise tel quel dans l'étape suivante. Point de fusion : 219-220,5°C.

### 5. 7-chloro-5-méthyl-4-oxo-3-phényl-3,5-dihydro-4H-pyridazino[4,5-b]indole-1-carboxaldéhyde.

A une solution de 3,3 g (9,7 mmoles) de 7-chloro-1-(hydroxyméthyl)-5-méthyl-3-phényl-3,5-dihydro-4*H-*pyridazino[4,5-*b*]indol-4-one dans 300 ml de dichlorométhane, on ajoute 5,7 g (65,6 mmoles) de dioxyde de manganèse et on agite le mélange réactionnel pendant 24 h à reflux.
On refroidit le milieu, on le filtre sur membrane Téflon™, on rince le solide avec du dichlorométhane, puis on concentre le filtrat sous pression réduite.
On isole 2,88 g (8,53 mmoles) de composé sous forme d'un solide blanc qu'on utilise tel quel dans l'étape suivante.
Point de fusion : 235-236°C.

### 6. 7-Chloro-5-méthyl-4-oxo-3-phényl-3,5-dihydro-4H-pyridazino[4,5-b]indole-1-acétonitrile.

A une solution de 2,14 g (10,96 mmoles) de 1-[(isocyanométhyl)sulfonyl]-4-méthylbenzène dans 50 ml de 1,2-diméthoxyéthane, on ajoute, par petites portions, 1,27 g (10,96 mmoles) de 1,1-diméthyléthylate de potassium, on agite le mélange réactionnel pendant 30 min à -60°C, on ajoute 2,88 g (8,53 mmoles) de 7-chloro-5-méthyl-4-oxo-3-phényl-3,5-dihydro-4*H*-pyridazino[4,5-*b*]indole-1-carboxaldéhyde et on agite le mélange réactionnel durant 3 h 30 min à -60°C. On ajoute 9 ml de méthanol et on agite encore le mélange réactionnel 30 min à température ambiante et 1 h à reflux.
On refroidit le milieu, on le concentre sous pression réduite, on ajoute au résidu de l'eau, 5 ml d'acide acétique et 200 ml de dichlorométhane, on décante la phase organique et on extrait la phase aqueuse avec du dichlorométhane. On réunit les phases organiques, on les lave à l'eau, on les sèche sur sulfate de sodium, on les filtre, on les concentre sous pression réduite et on purifie le résidu par chromatographie sur gel de silice.
On isole 1,87 g (5,36 mmoles) de composé sous forme d'un solide blanc qu'on utilise tel quel dans l'étape suivante.
Point de fusion : 305-315°C.

### 7. 7-Chloro-5-méthyl-4-oxo-3-phényl-3,5-dihydro-4H-pyridazino[4,5-b]indole-1-acétate de méthyle.

A une solution de 1,83 g (5,25 mmoles) de 7-chloro-5-méthyl-4-oxo-3-phényl-3,5-dihydro-4*H*-pyridazino[4,5-b]indole-1-acétonitrile dans 250 ml de méthanol, on ajoute du chlorure d'hydrogène jusqu'à saturation de la solution et on agite le mélange réactionnel pendant 4 h à reflux.
On refroidit le milieu, on concentre le mélange réactionnel sous pression réduite, on ajoute au résidu 25 ml d'eau et 25 ml de méthanol. Après agitation on recueille l'insoluble par filtration, on le lave à l'eau et à l'éther diéthylique, on le sèche et on le purifie par chromatographie sur colonne de gel de silice.
On isole 1,00 g (2,62 mmoles) de composé sous forme d'un solide blanc.
Point de fusion : 188,5-190°C.

### 8. 7-Chloro-N,N,5-triméthyl-4-oxo-3-phényl-3,5-dihydro-4H-pyridazino[4,5-b]indole-1-acétamide.

Sous argon, à une solution de 0,49 g (6 mmoles) de chlorhydrate de diméthylamine dans 80 ml de toluène, on ajoute, à 0°C, 3 ml (6 mmoles) de triméthylaluminium (2M dans le toluène) et on agite le mélange réactionnel pendant 1h 30 min à température ambiante.
On ajoute 0,21 g (0,55 mmoles) de 7-chloro-5-méthyl-4-oxo-3-phényl-3,5-dihydro-4*H*-pyridazino[4,5-*b*]indole-1-acétate de méthyle et on agite le mélange réactionnel pendant 6 h à reflux.
On refroidit le milieu à 4°C, on ajoute 10 ml d'eau et 100 ml de dichlorométhane, on filtre la solution, et on concentre le filtrat sous pression réduite.
On ajoute au résidu de l'eau, de d'acide chlorhydrique 1M et 150 ml de dichlorométhane, on sépare la phase organique, on la lave à l'eau, on la sèche sur sulfate de sodium, on la filtre, on la concentre sous pression réduite et on purifie le résidu par chromatographie sur colonne de gel de silice.
Après recristallisation dans un mélange de dichlorométhane et d'acétate d'éthyle, on isole 0,2 g (0,51 mmoles) de composé sous forme d'un solide blanc à l'aspect cotonneux.
Point de fusion : 229,5-230°C.

Le tableau qui suit illustre les structures chimiques et les propriétés physiques de quelques composés utilisables selon l'invention.

### Légende du tableau

"Me" et "Et", désignent, respectivement, un groupe méthyle et éthyle.
"pyrrolid", "piperid" et "morph" désignent, respectivement, un groupe pyrrolidinyle, pipéridinyle et morpholinyle.

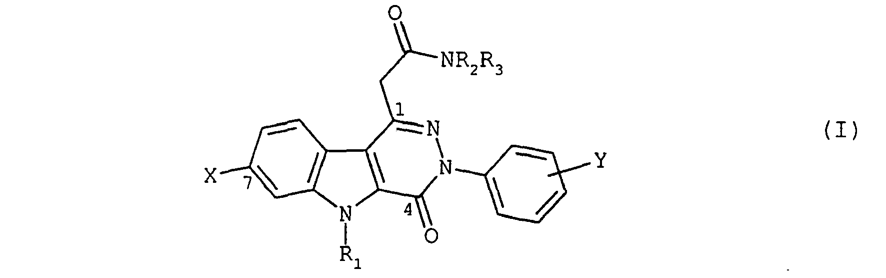

**Tableau**

| N° | X | Y | R₁ | NR₂R₃ | F (°C) |
|---|---|---|---|---|---|
| 1 | Cl | H | Me | NMe₂ | 229,5-230 |
| 2 | Cl | H | Me | NEt₂ | 167-168 |
| 3 | Cl | H | Me | pyrrolid | 260-263 |
| 4 | Cl | H | Me | morph | 273,5-274,5 |
| 5 | Cl | 3-Me | Me | NMe₂ | 204-205,5 |
| 6 | Cl | 3-Me | Me | NEt₂ | 200,5-201 |
| 7 | Cl | 3-Me | Me | pyrrolid | 268-269,5 |
| 8 | Cl | 3-Cl | Me | NMe₂ | 231-232 |
| 9 | Cl | 3-Cl | Me | NEt₂ | 202,5-203 |
| 10 | Cl | 3-Cl | Me | pyrrolid | 257-258,5 |
| 11 | Cl | 3-Cl | Me | piperid | 218-219 |
| 12 | Cl | 2-Cl | Me | NMe₂ | 253-255 |
| 13 | Cl | 2-Cl | Me | NEt₂ | 206-208 |
| 14 | Cl | 2-Cl | Me | pyrrolid | 295-297 |
| 15 | Cl | 4-Cl | Me | NMe₂ | 235-237 |
| 16 | Cl | 4-Cl | Me | NEt₂ | 223,5-224,5 |
| 17 | Cl | 4-Cl | Me | pyrrolid | 265-266 |
| 18 | Cl | 3-OMe | Me | NMe₂ | 200,5-202,5 |
| 19 | Cl | 3-OMe | Me | NEt₂ | 201-202 |
| 20 | Cl | 3-OMe | Me | pyrrolid | 240-242 |
| 21 | Cl | 3-NO₂ | Me | NMe₂ | 275-277,5 |
| 22 | Cl | 3-NO₂ | Me | NEt₂ | 228-228, 5 |
| 23 | Cl | 3-NO₂ | Me | pyrrolid | 261-263 |
| 24 | Cl | 3-F | Me | NMe₂ | 225-226,5 |
| 25 | Cl | 3-F | Me | NEt₂ | 171-172 |
| 26 | Cl | 3-F | Me | pyrrolid | 270-271,5 |
| 27 | Cl | 3,5-(Cl)₂ | Me | NEt₂ | 239-240,5 |
| 28 | Cl | 4-Cl | Me | NMeEt | 216, 5-217,5 |
| 29 | Cl | H | Me | NHMe | 305-307 |
| 30 | Cl | H | Me | NH₂ | 292-293 |
| 31 | Cl | 4-OMe | Me | NMe₂ | 233-234 |
| 32 | Cl | 4-OMe | Me | NEt₂ | 172-174 |
| 33 | Cl | 4-OH | Me | NMe₂ | 298-300 |
| 34 | Cl | 4-OH | Me | NEt₂ | 271-272 |

Les protocoles et les résultats des essais qui ont été réalisés sont décrits ci-après.

### Etude de la liaison [³H]Ro5-4864 aux récepteurs de type - périphérique aux benzodiazépines.

L'affinité des composés de l'invention pour récepteurs de type périphérique aux benzodiazépines (site p, ou PBR) a été déterminée.

Les récepteurs sites p peuvent être marqués sélectivement dans des membranes de rein de rat incubées en présence de [³H]Ro5-4864. Les composés ont fait l'objet d'une étude *in vitro* quant à leur affinité pour ces récepteurs.
Les animaux utilisés sont des rats mâles Sprague Dawley (Iffa Credo) de 180 à 300 mg. Après décapitation, on prélève le rein et le tissu est homogénéisé à 4°C au moyen d'un homogénéiseur Polytron™ pendant 2 min à 6/10 de la vitesse maximale dans 35 volumes de tampon phosphate Na₂HPO₄ 50 mM à un pH ajusté à 7,5 avec du NaH₂PO₄. L'homogénat membranaire est filtré sur gaze et dilué 10 fois avec du tampon.
Le [³H]Ro5-4864 (Activité spécifique : 70-90 Ci/mmole ; New England Nuclear), à une concentration de 0,5 nM, est incubé en présence de 100 µl de l'homogénat membranaire dans un volume final de 1 ml de tampon contenant le composé à tester.
Après une incubation de 3 h à 0°C, on récupère les membranes par filtration sur filtres Whatman GF/B™ qu'on lave avec 2 fois 4,5 ml de tampon d'incubation froid (0°C). On mesure la quantité de radioactivité retenue par le filtre par scintigraphie liquide.
Pour chaque concentration de composé étudié, on détermine le pourcentage d'inhibition de la liaison du [³H]Ro5-4864, puis la concentration CI₅₀, concentration qui inhibe 50% de la liaison spécifique.
Les CI₅₀ des composés les plus actifs vont de 0,6 nM à 20 nM.

Les composés utilisables selon l'invention sont, en conséquence, des ligands à haute affinité pour les récepteurs de type périphérique aux benzodiazépines.

### Etude de l'activité neurotrophe.

### Test de régénération du nerf facial lésé par mesure de la récupération fonctionnelle du réflexe palpébral, selon une modification de la méthode de K. Kujawa et al., Experimental Neurology (1989) 105 80-85.

La lésion du nerf facial par congélation locale entraîne une dégénérescence de la partie distale du nerf facial et une perte de la fonction du clignement de la paupière.
Les produits à étudier sont administrés par voie intrapéritonéale ou orale 2 fois par jour avec un décalage de 6 à 8 h, tous les jours pendant 10 jours (durée de l'expérience). Le premier traitement est administré 30 min avant la lésion.
Observation des animaux : la récupération de la fonction des paupières chez les animaux lésés est observée tous les jours, une fois le matin de J0 à J5 et 2 fois (matin et soir avec 6 à 8 h de décalage) de J6 à J10, avant chaque traitement, selon un score théorique allant de 0 jusqu'à 4.
Score 0 : oeil ouvert, score 1 : oeil fermé avec un degré inférieur à la moitié de l'oeil ; score 2 : degré de fermeture compris entre 1/2 et 3/4 ; score 3 : degré de fermeture supérieur à 3/4 ; score 4 : oeil complètement fermé.
Les résultats sont exprimés par le rapport des AUC ("area under the curve") du groupe traité et du groupe témoin.
Les rapports d'AUC des composés les plus actifs se situent entre 1,12 et 1,20.
Ces composés augmentent donc de 12 à 20% la récupération du réflexe palpébral après lésion du nerf facial.

### Test de survie des motoneurones après section du nerf

### facial chez le rat âgé de 4 jours.

Après lésion du nerf facial chez le rat immature, les motoneurones du noyau facial subissent une mort neuronale par apoptose. L'évaluation de la survie neuronale est réalisée à l'aide de méthodes histologiques et comptage neuronal.
Des rats immatures de 4 jours sont anesthésiés au pentobarbital (3 mg/kg par voie i.p.). Le nerf facial droit est dégagé et sectionné, à sa sortie du foramen stylomastoïdien. Après le réveil, les ratons sont remis avec leur mère et traités, pendant 7 j, par une ou deux administrations quotidiennes, par voie orale ou intrapéritonéale, à des doses allant de 1 à 10 mg/kg. 7 j après la lésion, les animaux sont décapités, et les cerveaux congelés dans l'isopentane à -40°C. Le noyau facial est coupé au cryostat, en sections de 10 µm, dans sa totalité. Les motoneurones sont colorés au crésyl violet et comptés à l'aide du logiciel Histo™ (Biocom™).
Dans ce modèle, les composés les plus actifs augmentent la survie neuronale d'environ 10 à 30%. A titre d'exemple, le composé décrit dans l'Exemple (N°1 du tableau) augmente la survie neuronale de 31% par voie i.p.

Les résultats des essais décrits ci-dessus montrent que les composés de formule générale (I) favorisent la régénération nerveuse.

### Etude des effets cardioprotecteurs.

Les effets cardioprotecteurs ont été étudiés sur le coeur isolé de lapin soumis à une ischémie régionale et à une reperfusion. La taile de l'infarctus ainsi que la récupération de la fonction contractile à la reperfusion ont été mesurées.

Des lapins néo-zélandais (2,3 à 2,5 kg, ESD France) sont anesthésiés par un mélange kétamine-xylasine et héparinés.

Le coeur est prélevé et rapidement perfusé par voie aortique rétrograde à une pression de 75 mmHg avec une solution de type Krebs Henselheit. Un ballonnet est introduit dans le ventricule gauche et une pression télédiastolique de 5 mmHg est imposée. Après une période de stabilisation de 20 min, le composé à étudier (1 mM) ou le véhicule est ajouté dans la solution de perfusion 15 min avant l'ischémie et pendant toute la durée de l'expérience. L'ischémie régionale est créée par ligature totale de l'artère coronaire gauche pendant 30 min, le coeur étant ensuite reperfusé pendant 2 h.
Les paramètres de pression ventriculaire, de fréquence cardiaque et de débit coronaire sont suivis pendant toute l'expérience.
A la fin de la reperfusion, l'artère coronaire est de nouveau occluse et de l'encre de Chine est perfusée afin de délimiter la zone à risque. Des coupes transversales sont alors réalisées et incubées dans une solution de triphényltétrazolium à 1%, afin de mesurer la taille de l'infarctus. La quantification de la zone de nécrose, exprimée en % de la zone à risque, est obtenue grâce à un logiciel d'analyse d'images.
A titre d'exemple, le composé décrit dans l'Exemple (N°1 du tableau) entraine une réduction significative de la taille de l'infarctus de 47% (témoins : 41,7 ± 5,3 vs composé étudié : 22 ± 3,3 ; p<0,01). Les zones à risque d'environ 50% sont comparables dans les deux groupes.
Le composé N°1 diminue la contracture à la reperfusion et restaure significativement la pression ventriculaire gauche (pourcentage de de récupération pré-ischémique de 36% après 2 h de reperfusion chez les témoins contre 65% chez les animaux traités), les dP/dt max et min et le double produit fréquence-pression.

### Etude des effets rénoprotecteurs.

L'expérience est réalisée sur des rats mâles Sprague-Dawley (Charles River France) de 270 à 330 g. Les animaux sont anesthésiés au pentobarbital (60 mg/kg i.p.), intubés et ventilés artificiellement, leur température est maintenue entre 37 et 38°C.
Un laparotomie de 3 cm est réalisée sur l'animal en décubitus dorsal, les artères rénales droite et gauche sont dégagées et occluses pendant 30 min, puis les reins sont reperfusés sous contrôle visuel et l'incision est refermée. Les taux sériques de créatinine et d'azote uréique sont déterminés à partir d'échantillons de sang prélevés au niveau du sinus orbitaire, à J0 (avant l'anesthésie), puis à J1, J2, J3, J4, J8 (après l'occlusion-reperfusion).
Le composé à étudier, ou son véhicule (Tween 80 à 2%) est administré à la dose de 3 mg/kg par voie i.p. 60 min avant l'occlusion.
A titre d'exemple, le composé N°1 réduit la créatininémie de 56% et l'urémie de 49%, en comparaison du véhicule à J3. Il réduit également la mortalité des animaux : 1/12 contre 4/9 chez les animaux qui ont reçu seulement le véhicule.

Les deux essais qui précèdent montrent, d'une part, que les composés utilisables selon l'invention réduisent la taille de l'infarctus induit par ischémie-reperfusion cardiaque chez le lapin et permet une meilleure récupération de la fonction contractile à la reperfusion, et, d'autre part, qu'ils limitent l'insuffisance rénale aiguë provoquée par un épisode d'ischémie-reperfusion rénale.

Les composés de formule générale (I) peuvent donc être utilisés pour la préparation de médicaments destinés à la prévention et au traitement des neuropathies périphériques de différent types, comme les neuropathies traumatiques ou ischémiques, neuropathies infectieuses, alcooliques, médicamenteuses ou génétiques, ainsi que des affections du motoneurone, telle que les amyotrophies spinales et la sclérose latérale amyotrophique.

Les composés utilisables selon l'invention peuvent aussi être utilisés dans les traitements de l'insuffisance rénale aiguë ou chronique, de la glomérulonéphrite, de la néphropathie diabétique, de l'ischémie et de l'insuffisance cardiaques, de l'infarctus du myocarde, de l'ischémie des membres inférieurs, du vasospasme coronaire, de l'angine de poitrine, des pathologies associées aux valves cardiaques, des maladies cardiaques inflammatoires, des effets secondaires dus à des médicaments cardiotoxiques ou aux suites d'une chirurgie cardiaque, de l'athérosclérose et de ses complications thrombo-emboliques, de la resténose, des rejets de greffes, des conditions liées à une prolifération ou une migration incorrectes des cellules musculaires lisses.

Ainsi la présente invention a pour objet l'utilisation des composés de formule générale (I) pour la préparation de compositions pharmaceutiques contenant une dose efficace d'au moins un composé de formule générale (I), à l'état de base ou de sel ou de solvat pharmaceutiquement acceptable, et en mélange, le cas échéant, avec des excipients convenables.
Lesdits excipients sont choisis selon la forme pharmaceutique et le mode d'administration souhaité.
Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.
Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,001 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides
ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports
ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Utilisation d'un composé de formule générale (I) 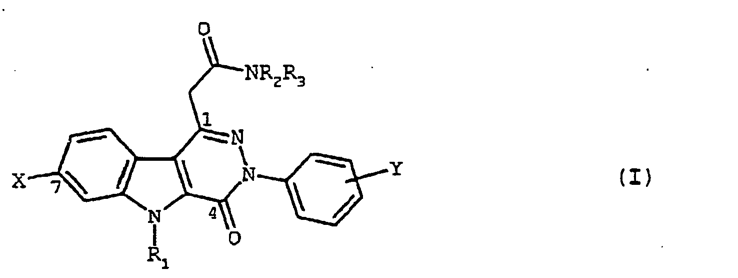 dans laquelle
X représente un atome d'halogène,
Y représente un ou plusieurs atomes ou groupes choisis parmi l'hydrogène, les halogènes et les groupes hydroxy, méthyle, méthoxy et nitro,
R₁ représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle ou morpholinyle,
pour la préparation d'un médicament destiné à la prévention et au traitement des maladies et troubles liés au dysfonctionnement des récepteurs de type périphérique aux benzodiazépines et choisis parmi les maladies dégénératives du système nerveux périphérique, les maladies et troubles cardiaques, et les néphropathies.

2. Utilisation selon la revendication 1, **caractérisée en ce que** le composé de formule générale (I) est le 7-chloro-*N,N,*5-triméthyl-4-oxo-3-phényl-3,5-dihydro-4*H*-pyridazino-[4,5-*b*]indole-1-acétamide.

3. Utilisation selon l'une des revendications 1 et 2, **caractérisée en ce que** la maladie ou trouble est une maladie dégénérative du système nerveux périphérique.

4. Utilisation selon l'une des revendications 1 et 2, **caractérisée en ce que** la maladie ou trouble est une maladie ou trouble cardiaque.

5. Utilisation selon l'une des revendications 1 et 2, **caractérisée en ce que** la maladie ou trouble est une néphropathie.

## Claims

1. Use of a compound of general formula (I) 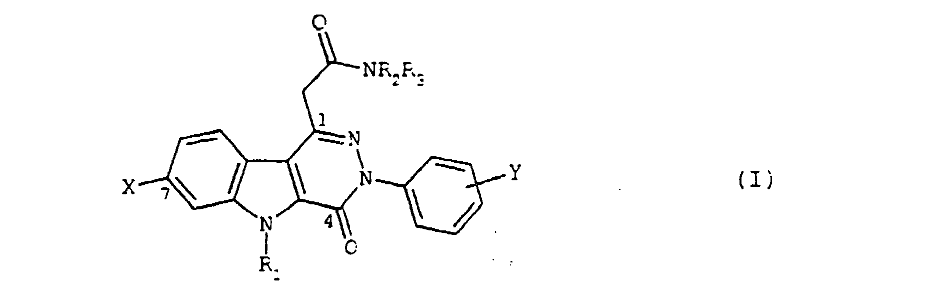 in which
X represents a halogen atom,
Y represents one or more atoms or groups chosen from hydrogen, halogens and hydroxyl, methyl, methoxy and nitro groups,
R₁ represents a (C₁-C₄)alkyl group,
R₂ and R₃ each represent, independently of each other, a hydrogen atom or a (C₁-C₄)alkyl group, or alternatively R₂ and R₃ form, with the nitrogen atom carrying them, a pyrrolidinyl, piperidinyl or morpholinyl group,
for the preparation of a medicament for the prevention and treatment of diseases and disorders linked to the dysfunctioning of peripheral-type benzodiazepine receptors and chosen from the degenerative diseases of the peripheral nervous system, cardiac diseases or disorders, and nephropathies.

2. Use according to Claim 1, **characterized in that** the compound of general formula (I) is 7-chloro-*N*,*N*,5-trimethyl-4-oxo-3-phenyl-3,5-dihydro-4*H*-pyridazino[4,5-*b*]indole-1-acetamide.

3. Use according to either of Claims 1 and 2, **characterized in that** the disease or disorder is a degenerative disease of the peripheral nervous system.

4. Use according to either of Claims 1 and 2, **characterized in that** the disease or disorder is a cardiac disease or disorder.

5. Use according to either of Claims 1 and 2, **characterized in that** the disease or disorder is a nephropathy.

## Patentansprüche

1. Verwendung einer Verbindung der allgemeinen Formel (I) 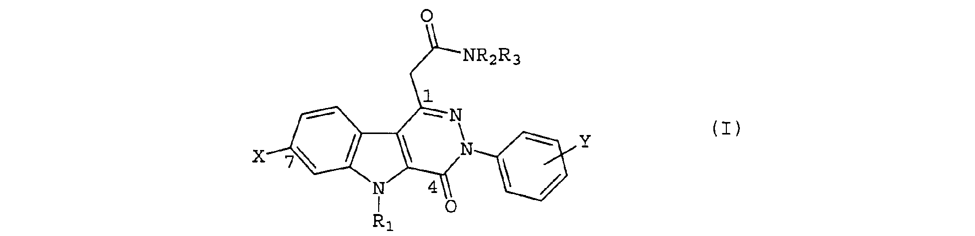 in der
X ein Halogenatom darstellt,
Y ein oder mehrere Atome oder Gruppen darstellt, ausgewählt unter Wasserstoff, den Halogenen und den Gruppen Hydroxy, Methyl, Methoxy und Nitro,
R₁ eine Gruppe (C₁-C₄)-Alkyl darstellt,
R₂ und R₃ jeweils unabhängig voneinander ein Wasserstoffatom oder eine Gruppe (C₁-C₄)-Alkyl darstellen, oder auch R₂ und R₃ zusammen mit dem Stickstoffatom, das sie trägt, eine Gruppe Pyrrolidinyl, Piperidinyl oder Morpholinyl bilden,
zur Herstellung eines Arzneimittels, das vorgesehen ist für die Vorbeugung und Behandlung von Erkrankungen und Störungen, die mit Funktionsstörungen der Benzodiazepin-Rezeptoren vom peripheren Typ verbunden sind und ausgewählt werden unter den degenerativen Erkrankungen des peripheren Nervensystems, den Herzerkrankungen und Herzstörungen und den Nephropathien.

2. Verwendung nach Anspruch 1, **dadurch gekennzeichnet, daß** die Verbindung der allgemeinen Formel (I) das 7-Chlor-N,N,5-trimethyl-4-oxo-3-phenyl-3,5-dihydro-4H-pyridazino-[4,5-b]-indol-1-acetamid ist.

3. Verwendung nach einem der Ansprüche 1 und 2, **dadurch gekennzeichnet, daß** die Erkrankung oder Störung eine degenerative Erkrankung des peripheren Nervensystems ist.

4. Verwendung nach einem der Ansprüche 1 und 2, **dadurch gekennzeichnet, daß** die Erkrankung oder Störung eine Herzerkrankung oder Herzstörung ist.

5. Verwendung nach einem der Ansprüche 1 und 2, **dadurch gekennzeichnet, daß** die Erkrankung oder Störung eine Nephropathie ist.
